# EUROPEAN PATENT APPLICATION

(11) **EP 4 491 724 A1**
(43) Date of publication of application: **15.01.2025**
(21) Application number: 23766819.9
(22) Date of filing: 07.03.2023
(51) Int. Cl.: C12N 11/00, C12N 9/90, C12N 15/61, C12R 1/06

(54) **DRIED BODY OF IMMOBILIZED ENZYME CAPABLE OF ENDURING DRYING, AND MANUFACTURING METHOD AND PRESERVATION METHOD THEREFOR**

(30) Priority: 07.03.2022 JP 2022034030
(71) Applicant: National University Corporation Kagawa University, Takamatsu-shi, Kagawa 760-8521 (JP)
(72) Inventor: YOSHIHARA, Akihide, Takamatsu-shi, Kagawa 760-8521 (JP); IZUMORI, Ken, Takamatsu-shi, Kagawa 760-8521 (JP); KATO, Shiro, Takamatsu-shi, Kagawa 760-8521 (JP); MOCHIZUKI, Susumu, Takamatsu-shi, Kagawa 760-8521 (JP); AKIMITSU, Kazuya, Takamatsu-shi, Kagawa 760-8521 (JP); YOSHIDA, Hiromi, Takamatsu-shi, Kagawa 760-8521 (JP)
(74) Representative: TBK
(86) International application number: PCT/JP2023/008469
(87) International publication number: WO 2023/171643

(57) **Abstract**

[Problem] To provide a ketose 3-epimerase or a protein having the same activity in a form in which an effect of the enzyme is maintained for a long period of time even under severe preservation conditions.

[Solution] A dried body of an immobilized enzyme or an immobilized protein having enzymatic activity thereof, which is an enzyme capable of enduring drying, wherein the enzyme or the protein having enzymatic activity thereof is a ketose 3-epimerase or a protein having ketose 3-epimerase activity, and the immobilized enzyme prior to drying or the immobilized protein having enzymatic activity thereof prior to drying is an immobilized enzyme or an immobilized protein having enzymatic activity thereof, in which a temperature (an optimum temperature) indicating maximum activity has further increased as compared with prior to immobilization. A manufacturing method including immobilizing the enzyme or the protein, manufacturing an immobilized enzyme or an immobilized protein having enzymatic activity of the enzyme, in which a temperature (an optimum temperature) indicating maximum activity has further increased as compared with prior to immobilization, and performing drying. The enzyme or the protein is supported by a carrier which is an ion exchange resin, an organic polymer carrier, and/or an inorganic carrier, and is dried.

## Description

### TECHNICAL FIELD

The present invention relates to a dried body of an immobilized enzyme or an immobilized protein having enzymatic activity thereof, which is a ketose 3-epimerase (for example, D-allulose 3-epimerase) or a protein having ketose 3-epimerase activity capable of enduring drying, in which transportation resistance is maintained and a decrease in activity is suppressed, a manufacturing method and preservation method therefor.

### BACKGROUND ART

In the prior art, pure rare sugar D-allulose (D-psicose) is produced using a ketose 3-epimerase (for example, D-allulose 3-epimerase) with D-fructose as a raw material. The rare sugar D-allulose can be produced as a powdered crystal by acting an epimerase on D-fructose to produce a mixture of D-fructose and D-allulose, and separating and purifying the mixture. The D-allulose itself has applications as a sweetener, a pharmaceutical product, and the like, and has started to mass-produce and sell worldwide. Since the D-allulose has zero energy and various functions, it is sold as a sweetener in particular.

Furthermore, in recent years, the problem of climate change, which is a problem that humanity is facing, is increasing in importance as a specific issue of "decarbonization" in the food and beverage industry related to starch, which is a carbon assimilation product of plants, and there is a demand for the development of rare sugar products related to starch that can compete in the world.

As one of the reasons, in order to smoothly introduce the product into the world market by suppressing the raw material cost, which accounts for a majority of the manufacturing cost, it is considered that, for example, and the rare sugar is improved to have the functionality of the rare sugar by introducing the rare sugar into the inexpensive isomerized sugar that is a mixed sugar including D-glucose and D-fructose, in which in 1999, the consumption of isomerized sugar in the United States increased to 14 million tons, and the industry thereof already exists in a global scale. For example, it is considered that a mass production method (new manufacturing process) on a worldwide scale is directed to obtain a sweetener having a flavor close to that of sugar, which has a functionality of D-allulose and contains three types of sugars consisting of D-glucose, D-fructose, and D-allulose.

The isomerized sugar (high-fructose corn syrup (HFCS)) used as the above-described inexpensive raw material has been developed in Japan in the 1960s and then has commercially produced in the United States. The initially manufactured HFCS42 (having a fructose content of 42%) has not been consumed in large amounts because of the reason that the sweetness was insufficient, but in 1970s, HFCS55 (having a fructose content of 55%) having a stronger sweetness has been developed, and a major beverage manufacturer has started to use the HFCS55 as a substitute for sugar because it has been cheaper than sugar. Thereafter, with the expansion of consumption of soft drinks, the consumption of HFCS55 also increased, and in 1999, the consumption of isomerized sugar in the United States increased to 14 million tons.

Today, the isomerized sugar market in the United States is already matured, and it can be said that the demand is in a saturated state. The production amount of HFCS42 is decreased with the decrease in the consumption in the United States, and the net import amount (the amount obtained by subtracting the export amount from the import amount) is also very small. In recent years, the decrease in the consumption amount has been moderate, but there is no sign of a reversal and an increase trend.

The domestic consumption in United States of HFCS55 is also on a decreasing trend, but a certain stable production amount is maintained due to the export demand to Mexico. However, in addition to the fact that there is no element that indicates that the domestic consumption in United States increases reversely in the future as in the case of HFCS42, the isomerized sugar market in Mexico has recently been saturated. Therefore, it is difficult to continue the situation in which the decrease in domestic demand is covered by the increase in export and the production amount is stabilized as a result.

The costs required for the manufacture of isomerized sugar include costs of raw materials (corn), labor costs, fuel costs, and the like, and the so-called "production cost" is obtained by subtracting income from by-products (gluten meal, gluten feed, and corn oil) obtained in the isomerized sugar manufacture process.

Although the production cost of HFCS55 is higher than that of HFCS42 by 50 to 60 US dollars (6,150 to 7,380 yen) on average per ton, the raw material cost is a factor for fluctuation of the production cost in both HFCS42 and HFCS55. Therefore, the production costs of HFCS42 and HFCS55 are moving in substantially the same manner.

While the expenses such as labor costs, fuel costs, and electricity costs are stable, but in recent years, the price of corn has fluctuated greatly. Therefore, the production cost of isomerized sugar has also changed greatly, for example, the production cost was the highest in 2012 and decreased by about 30% in the following 2 years.

D-Allulose is also referred to as D-psicose, is an epimer of D-fructose, has a sweetness degree of about 70% of that of sugar, and is similar to D-fructose in the quality of sweetness. However, unlike D-fructose, D-allulose is hardly metabolized during absorption in the body, has a calorie close to zero, and has been clarified to have a function of reducing abdominal fat by inhibiting the activity of lipid synthesis enzyme. So far, it has been reported that D-allulose can be used as a low-calorie sweetener (Patent Document 1), as a sweetener effective for weight loss (Non-patent Documents 1 and 2). Patent Document 2 describes that an effect of a blood glucose elevation suppressant of D-allulose is focused on, and D-allulose is used for health foods, foods and beverages for diabetes patients, foods and beverages for slimming, and the like.

In addition, D-tagatose has an energy of about 2 kcal/g with a sweetness degree of about 90% of sugar, and is used as a low-calorie sweetener. Furthermore, it has been found that the D-tagatose has an effect of increasing HDL cholesterol and an effect of suppressing the accumulation of body fat (Patent Document 3), and also has the effect of a postprandial blood glucose elevation suppressant as D-allulose has.

Currently, an isomerase-based method is used for the manufacture of these rare sugars, which is a result of finding a useful enzyme reaction. The ketose 3-epimerase, which is one type of the isomerase, can have a plurality of ketoses as a substrate, and among the ketoses as the substrate, an enzyme having the highest epimerization activity at a 3-position is named after the ketose, and for example, an enzyme having the highest epimerization activity at a 3-position with respect to D-tagatose is called D-tagatose 3-epimerase. By using this D-tagatose 3-epimerase (DTE), a technique for manufacturing D-allulose, which is a rare sugar, from D-fructose has been established.

For example, Non Patent Document 3 discloses a D-ketose 3-epimerase derived from a Pseudomonas cichorii ST-24 strain, and describes that D-allulose can be manufactured from D-fructose by using this enzyme. However, as the enzyme is also referred to as D-tagatose 3-epimerase, the epimerization activity at the 3-position of D-tagatose is the highest, and the activity on D-fructose is relatively weak.

In addition, Patent Document 4 has reported a method for producing D-psicose (D-allulose) using D-psicose 3-epimerase derived from Agrobacterium tumefaciens, and Patent Document 5 has reported a manufacturing method of D-allulose using a ketose 3-epimerase derived from Arthrobacter globiformis. Currently, Arthrobacter globiformis is used in a production site, but in order to mass-produce D-allulose or D-tagatose on an industrial scale, it is required to use a more active ketose 3-epimerase.

In such a case, a novel ketose 3-epimerase has been found, which is derived from a microorganism acceptable to use in a case of manufacturing food and known to be non-toxic, has high activity and heat resistance, and can catalyze isomerization from D-fructose to D-allulose at high activity (Patent Document 6).

### Citation List

### Patent Documents

[Patent Document 1] Japanese Patent No. 4473980
[Patent Document 2] Japanese Patent No. 5421512
[Patent Document 3] Japanese Patent No. 5749880
[Patent Document 4] Japanese Patent No. 4648975
[Patent Document 5] Japanese Patent No. 5997693
[Patent Document 6] International Publication No. WO2020/105709 Non-Patent Documents

[Non-Patent Document 1] Asia Pac. J. Clin. Nutr. (2001) 10, 233-237
[Non-Patent Document 2] Asia Pac. J. Clin. Nutr. (2004) 13, 127
[Non-Patent Document 3] Biosci. Biotech. Biochem. (1993) 57, 1037-1039

### SUMMARY

### Technical Problem

In the production of isomerized sugar, the raw material cost often accounts for a large proportion of the manufacturing cost, and the proportion of the merits of improving the enzyme catalytic performance is not large. However, in a case where the competition is at a world level, the business scale is very large, and the profit of improving the enzyme catalytic performance is large. Based on the heat resistance of the ketose 3-epimerase in Patent Document 6, it is expected that the use of the ketose 3-epimerase will be accelerated on a global scale, and the possibility of constructing a catalyst business or a licensing business for the worldwide supply of the enzyme is increasing.

As the business scale increases and production bases increase in various countries, there is a high possibility that there is a demand for a highly active enzyme catalyst ketose 3-epimerase and that transportation across countries from a specialized department of enzyme catalysts will start. In that case, in order to be able to use the enzyme catalysts as it is in the raw material injection part of the resin column upon arrival at the production site, it is appropriately considered to transport in the form of the immobilized enzyme, and a preservation method of related art of the immobilized enzyme is adopted, generally such as performing the immobilized enzyme immersed in an appropriate buffer solution at a low temperature (2°C to 15°C) without freezing, or adding an additive to prevent a decrease in activity.

However, in such a form, a new problem arises. There is an urgent need to solve problems such as the need to carry water in a case of long-distance transportation, the need for a dedicated operator for temperature management, the need to take a place for preservation, and the need for strict packaging to prevent water leakage. At the same time, the buffer solution itself is likely to be a suitable culture medium for microorganisms or the like, and the immobilized enzyme itself may be destroyed because of contamination by mold or the like. Furthermore, in recent years, the container transportation route between China and the United States has changed suddenly, and the transportation cost from Japan has soared, and the problem of different quality, that is, the transportation cost, has become more important.

It is clear that the dried immobilized enzyme is advantageous in order to solve the problems of (a) the price, (b) the required number of days, and (c) the management temperature, which are elements for selecting the transportation means.

Furthermore, it has been reported that Japan is between the container transportation routes between China and the United States has been standard, but the transportation from the United States to China is made by air, not stopping at Japan because it takes time to load, thereby the transportation cost from Japan is soaring. Under such a container shortage situation, it is necessary to realize efficient transportation while maintaining simplicity.

On the other hand, regarding as the scientific principle that "drying" is "stabilizing" to maintain the effect for a long period of time even under severe preservation conditions, in a complex structural body, the tertiary structure of the enzyme in the solution does not change significantly and the enzymatic activity can be maintained, but it is feared that the structure of the enzyme changes significantly while drying, and as a result, the activity of the enzyme is significantly reduced, and it is considered that the (freezing) drying operation itself can often cause deterioration. It is necessary to derive an answer to whether or not the principle can apply to the enzyme catalyst ketose 3-epimerase, particularly the immobilized enzyme.

The object of the present invention is to provide a ketose 3-epimerase (for example, D-allulose 3-epimerase) as an enzyme catalyst or a protein having ketose 3-epimerase activity in a form in which an effect of thereof is maintained for a long period of time even under severe preservation conditions, that is, a low-cost enzyme catalyst derived from high durability (lifetime) of the enzyme as an "enzyme capable of enduring drying", in order to spread a mass production method on a worldwide scale with sufficient price competitiveness, of a sweetener that contains three types of sugars of D-glucose, D-fructose, and D-allulose, has functionality of D-allulose, and has a flavor similar to that of sugar, by combining the use of, for example, "inexpensive isomerized sugar or inverted sugar, which is a mixed sugar containing D-glucose and D-fructose", as a rare sugar product that can compete in the world, for example, in term of a raw material cost occupying most of a manufacturing cost, the shortened multi-step process by production without using pseudo-moving layer chromatography, and the use of low-cost enzyme catalyst.

### Solution to Problem

The present invention discloses to provide a ketose 3-epimerase (for example, D-allulose 3-epimerase) or a protein having ketose 3-epimerase activity in a form of a dried body of an immobilized enzyme or an immobilized protein, in which an effect thereof is maintained for a long period of time even under severe preservation conditions.

The immobilized enzyme itself is obtained by using a biological molecule, and is an enzyme in which a decrease in activity cannot be avoided in a case of long-term use. Water as a solvent is a medium which allows to uniformly disperse various substances, and is also a medium which allows physical and chemical changes (deterioration) in the enzyme catalyst. In a case where the enzyme catalyst is in a dried state, even in a case where an unstable substance coexists, the unstable substance is solidified in the same manner as the enzyme catalyst. Therefore, movement of a substance (molecule) related to deterioration is also restricted, and the enzyme catalyst can be preserved for a long period of time. It can be said that the drying in this manner also contributes to the stability, but the enzyme molecule, which has been originally stable in the presence of water molecules, can be unstable because of loss of hydrogen bonding by the dehydration operation of drying. In addition, for a complex structural body such as an enzyme catalyst, the (freezing) drying operation itself can often be a denaturation factor to which the complex structural body is considered to be subjected. In a case where the activity is reduced, the immobilized enzyme is naturally replaced, which results in a large economic loss.

In order to solve various problems, in a case where a type of immobilized enzyme in which a ketose 3-epimerase (for example, D-allulose 3-epimerase) itself is carried on an ion exchange resin or the like can be adopted for preservation in a dry state, this can be an effective measure against transportation costs, prevention of contamination due to leakage of the liquid, microbial contamination, and the like. However, the business itself of using a ketose 3-epimerase (for example, D-allulose 3-epimerase) has just started, the scale thereof is not yet large, and the problem of drying in such an immobilized enzyme form has not been recognized so far. Although the movement has started for increasing production bases in various countries in the world, there is no incentive to solve the problem of drying at all, and an immobilized dried body of a ketose 3-epimerase (for example, D-allulose 3-epimerase) has not been practically used in the related art. Regarding a ketose 3-epimerase (for example, D-allulose 3-epimerase), it is not known whether or not an immobilized dried body in which the transportation resistance is maintained and the decrease in activity is suppressed is obtained, until actually tried.

The present inventors first considered that the first step is to recognize the problem of drying in the immobilized enzyme from, intensively studied and conducted experiments, and have found that, in a case of the immobilized enzyme, although the enzyme molecule, which has been originally stable in the presence of water molecules, can be unstable because of loss of hydrogen bonding by the dehydration operation of drying, the enzyme molecule and the carrier molecule can form a hydrogen bond to each other by appropriately selecting a carrier, and the enzyme molecule can be stably present even in a case of low moisture content, that is, have found that in a case where, in the immobilized enzyme, the stability having at least the same degree of activity as that before immobilization at the same temperature or a temperature higher than that, the problem is solved, the enzyme (ketose 3-epimerase) or the protein having enzymatic activity thereof (protein having ketose 3-epimerase activity) is a "dried body in which washing prior use is not required, transportation resistance is maintained, and the decrease in activity is suppressed" also after drying. Therefore, the present invention has been completed.

The present invention relates to a dried body of an immobilized enzyme or an immobilized protein having enzymatic activity thereof, which is an enzyme capable of enduring drying, as described in the following (1) to (9) as a summary.
(1) A dried body of an immobilized enzyme or an immobilized protein having enzymatic activity thereof, which is an enzyme capable of enduring drying,
   wherein the enzyme or the protein having enzymatic activity thereof is a ketose 3-epimerase or a protein having ketose 3-epimerase activity, and
   the immobilized enzyme prior to drying or the immobilized protein having enzymatic activity thereof prior to drying is an immobilized enzyme or an immobilized protein having enzymatic activity thereof, in which a temperature (an optimum temperature) indicating maximum activity has further increased as compared with prior to immobilization.
(2) The dried body of an immobilized enzyme or an immobilized protein having enzymatic activity thereof, which is an enzyme capable of enduring drying according to (1),
   wherein the dried body is a dried body in which an additive for maintaining the enzymatic activity is not contained and the activity does not decrease for at least 6 months or more.
(3) The dried body of an immobilized enzyme or an immobilized protein having enzymatic activity thereof, which is an enzyme capable of enduring drying according to (1 ) or (2),
   wherein the dried body is a dried body of an enzyme or a protein having enzymatic activity thereof supporting by a carrier which is an ion exchange resin, organic polymer carrier, and/or an inorganic carrier.
(4) The dried body of an immobilized enzyme or an immobilized protein having enzymatic activity thereof, which is an enzyme capable of enduring drying according to any one of (1) to (3),
   wherein the dried body is a dried body in which washing prior use is not required, transportation resistance is maintained, and a decrease in activity is suppressed for at least 6 months or more.
(5) The dried body of an immobilized enzyme or an immobilized protein having enzymatic activity thereof, which is an enzyme capable of enduring drying according to any one of (1) to (4),
   wherein the dried body is a freeze-dried body or a 30°C-incubator-dried body.
(6) The dried body of an immobilized enzyme or an immobilized protein having enzymatic activity thereof, which is an enzyme capable of enduring drying according to any one of (1) to (5),
   wherein the enzyme or the protein having enzymatic activity thereof is selected from any one of the following enzyme (i) or protein having enzymatic activity thereof (ii),
   (i) a ketose 3-epimerase derived from a microorganism belonging to the genus Arthrobacter, which has a molecular mass of 36 kDa measured by SDS-PAGE and has the following substrate specificities of (A) and (B) and the following physicochemical properties of (C) and (D),
      (A) having activity of epimerizing a 3-position of a D- or L-ketose to generate a corresponding D- or L-ketose,
      (B) having highest epimerization activity at a 3-position of a D-allulose among D- or L-ketohexoses,
      (C) having a reaction optimum pH of 6 to 8,
      (D) having a reaction optimum temperature of 60°C,
   (ii) a protein described in the following (a), (b), (c), or (d),
      (a) a protein consisting of an amino acid sequence set forth in SEQ ID NO: 1,
      (b) a protein consisting of an amino acid sequence having a sequence identity of 75% or more with the amino acid sequence set forth in SEQ ID NO: 1, the amino acid sequence having no amino acid substitution at any sites of H6, S63, M110, E146, E152, D179, H182, H205, R211, or E240 of the amino acid sequence set forth in SEQ ID NO: 1, and maintaining a tertiary structure of the protein consisting of the amino acid sequence set forth in SEQ ID NO: 1, in which the protein has ketose 3-epimerase activity of the (A) and (B), and a ratio (T70/T50) of ketose 3-epimerase activity at a reaction temperature of 70°C to ketose 3-epimerase activity at a reaction temperature of 50°C is 1.55 or more,
      (c) a protein consisting of an amino acid sequence having a sequence identity of 75% or more with the amino acid sequence set forth in SEQ ID NO: 1, the amino acid sequence having no amino acid substitution at any sites of H6, S63, M110, E146, E152, D179, H182, H205, R211, or E240 of the amino acid sequence set forth in SEQ ID NO: 1, and maintaining the tertiary structure of the protein consisting of the amino acid sequence set forth in SEQ ID NO: 1, in which the protein has the ketose 3-epimerase activity of the (A) and (B), and residual activity is 31% or more after incubation at 60°C for 1 hour,
      (d) a protein consisting of an amino acid substitution mutant of the protein consisting of the amino acid sequence set forth in SEQ ID NO: 1, in which the protein has an amino acid substitution at at least one site of H56, T59, A60, P77, L94, L97, D101, A109, A128, V129, V132, S144, S167, P172, E199, A200, K202, or S218 of the amino acid sequence set forth in SEQ ID NO: 1, the protein has the ketose 3-epimerase activity of the (A) and (B), and the protein consists of a mutant having a higher ratio (T70/T50) of ketose 3-epimerase activity at a reaction temperature of 70°C to ketose 3-epimerase activity at a reaction temperature of 50°C or having a higher residual activity after incubation at 60°C for 1 hour as compared with the protein consisting of the amino acid sequence set forth in SEQ ID NO: 1.
(7) The dried body of an immobilized enzyme or an immobilized protein having enzymatic activity thereof, which is an enzyme capable of enduring drying according to any one of (1) to (6),
   wherein in the immobilized enzyme or the immobilized protein having enzymatic activity thereof, a crude enzyme present in a disrupted product of bacterial cell or a partially purified enzyme from a crude enzyme solution is immobilized on a carrier.
(8) The dried body of an immobilized enzyme or an immobilized protein having enzymatic activity thereof, which is an enzyme capable of enduring drying according to any one of (1) to (7),
   wherein substrate specificity of the ketose 3-epimerase is in the following order: D-allulose, D-tagatose, L-tagatose, D-fructose, and L-allulose.
(9) The dried body of an immobilized enzyme or an immobilized protein having enzymatic activity thereof, which is an enzyme capable of enduring drying according to any one of (6) to (8),
   wherein the microorganism belonging to the genus Arthrobacter is Arthrobacter histidinolovorans.

The present invention relates to a manufacturing method of a dried body of an immobilized enzyme or an immobilized protein having enzymatic activity thereof, which is an enzyme capable of enduring drying, as described in the following (10) to (18) as a summary.
(10) A manufacturing method of a dried body of an immobilized enzyme or an immobilized protein having enzymatic activity thereof, which is an enzyme capable of enduring drying,
   the manufacturing method comprising:
   immobilizing an enzyme ketose 3-epimerase or a protein having protein ketose 3-epimerase activity;
   manufacturing the immobilized enzyme or the protein having enzymatic activity of the enzyme in which a temperature (an optimum temperature) indicating maximum activity has further increased as compared with prior to immobilization; and
   performing drying.
(11) The manufacturing method of a dried body of an immobilized enzyme or an immobilized protein having enzymatic activity thereof, which is an enzyme capable of enduring drying according to (10),
   wherein the enzyme ketose 3-epimerase or the protein having ketose 3-epimerase activity is supported by and immobilized on a carrier which is an ion exchange resin, an organic polymer carrier, and/or an inorganic carrier.
(12) The manufacturing method of a dried body of an immobilized enzyme or an immobilized protein having enzymatic activity thereof, which is an enzyme capable of enduring drying according to (10) or (11),
   wherein the drying is performed without adding an additive for maintaining the enzymatic activity.
(13) The manufacturing method of a dried body of an immobilized enzyme or an immobilized protein having enzymatic activity thereof, which is an enzyme capable of enduring drying according to any one of (10) to (12),
   wherein the drying is performed by freeze-drying or incubator-drying at 30°C.
(14) The manufacturing method of a dried body of an immobilized enzyme or an immobilized protein having enzymatic activity thereof, which is an enzyme capable of enduring drying according to any one of (10) to (13),
   wherein the enzyme ketose 3-epimerase or the protein having ketose 3-epimerase activity is selected from any one of the following enzyme (i) or protein having enzymatic activity of the enzyme (ii),
   (i) a ketose 3-epimerase derived from a microorganism belonging to the genus Arthrobacter, which has a molecular mass of 36 kDa measured by SDS-PAGE and has the following substrate specificities of (A) and (B) and the following physicochemical properties of (C) and (D),
      (A) having activity of epimerizing a 3-position of a D- or L-ketose to generate a corresponding D- or L-ketose,
      (B) having highest epimerization activity at a 3-position of a D-allulose among D- or L-ketohexoses,
      (C) having a reaction optimum pH of 6 to 8,
      (D) having a reaction optimum temperature of 60°C,
   (ii) a protein described in the following (a), (b), (c), or (d),
      (a) a protein consisting of an amino acid sequence set forth in SEQ ID NO: 1,
      (b) a protein consisting of an amino acid sequence having a sequence identity of 75% or more with the amino acid sequence set forth in SEQ ID NO: 1, the amino acid sequence having no amino acid substitution at any sites of H6, S63, M110, E146, E152, D179, H182, H205, R211, or E240 of the amino acid sequence set forth in SEQ ID NO: 1, and maintaining a tertiary structure of the protein consisting of the amino acid sequence set forth in SEQ ID NO: 1, in which the protein has ketose 3-epimerase activity of the (A) and (B), and a ratio (T70/T50) of ketose 3-epimerase activity at a reaction temperature of 70°C to ketose 3-epimerase activity at a reaction temperature of 50°C is 1.55 or more,
      (c) a protein consisting of an amino acid sequence having a sequence identity of 75% or more with the amino acid sequence set forth in SEQ ID NO: 1, the amino acid sequence having no amino acid substitution at any sites of H6, S63, M110, E146, E152, D179, H182, H205, R211, or E240 of the amino acid sequence set forth in SEQ ID NO: 1, and maintaining the tertiary structure of the protein consisting of the amino acid sequence set forth in SEQ ID NO: 1, in which the protein has the ketose 3-epimerase activity of the (A) and (B), and residual activity is 31% or more after incubation at 60°C for 1 hour,
      (d) a protein consisting of an amino acid substitution mutant of the protein consisting of the amino acid sequence set forth in SEQ ID NO: 1, in which the protein has an amino acid substitution at at least one site of H56, T59, A60, P77, L94, L97, D101, A109, A128, V129, V132, S144, S167, P172, E199, A200, K202, or S218 of the amino acid sequence set forth in SEQ ID NO: 1, the protein has the ketose 3-epimerase activity of the (A) and (B), and the protein consists of a mutant having a higher ratio (T70/T50) of ketose 3-epimerase activity at a reaction temperature of 70°C to ketose 3-epimerase activity at a reaction temperature of 50°C or having a higher residual activity after incubation at 60°C for 1 hour as compared with the protein consisting of the amino acid sequence set forth in SEQ ID NO: 1.
(15) The manufacturing method of a dried body of an immobilized enzyme or an immobilized protein having enzymatic activity thereof, which is an enzyme capable of enduring drying according to any one of (10) to (14),
   wherein a crude enzyme present in a disrupted product of bacterial cell or a partially purified enzyme from a crude enzyme solution is immobilized on a carrier.
(16) The manufacturing method of a dried body of an immobilized enzyme or an immobilized protein having enzymatic activity thereof, which is an enzyme capable of enduring drying according to any one of (10) to (15),
   wherein substrate specificity of the ketose 3-epimerase is in the following order: D-allulose, D-tagatose, L-tagatose, D-fructose, and L-allulose.
(17) The manufacturing method of a dried body of an immobilized enzyme or an immobilized protein having enzymatic activity thereof, which is an enzyme capable of enduring drying according to any one of (14) to (16),
   wherein a microorganism belonging to the genus Arthrobacter is Arthrobacter histidinolovorans.
(18) The manufacturing method of a dried body of an immobilized enzyme or an immobilized protein having enzymatic activity thereof, which is an enzyme capable of enduring drying according to any one of (14) to (17),
   wherein a microorganism belonging to the genus Arthrobacter is Arthrobacter histidinolovorans Y586-1 strain deposited in Patent Microorganisms Depositary with accession number NITE BP-02813.

In addition, the present invention relates to a preservation method of a dried body of an immobilized enzyme or an immobilized protein having enzymatic activity thereof, which is an enzyme capable of enduring drying, in which transportation resistance is maintained and the decrease in activity is suppressed, as described in the following (19) and (20) as a summary.
(19) A preservation method of the dried body of an immobilized enzyme or an immobilized protein having enzymatic activity thereof, which is an enzyme capable of enduring drying according to any one of (1) to (9), the preservation method comprising:
   performing preservation at any one of a refrigerated temperature or a room temperature.
(20) The preservation method of the dried body of an immobilized enzyme or an immobilized protein having enzymatic activity thereof, which is an enzyme capable of enduring drying according to (19),
   wherein the dried body is sealed in a sealable container consisting of paper, plastic, or metal.

### Advantageous Effects of Invention

According to the present invention, a ketose 3-epimerase (for example, D-allulose 3-epimerase) or a protein having ketose 3-epimerase activity can be provided in a form in which an effect of the enzyme is maintained for a long period of time even under severe preservation conditions.

Therefore, it is possible to construct a catalyst business or a licensing business for worldwide supply of the enzyme, and to spread a mass production method on a worldwide scale with sufficient price competitiveness, of a sweetener that contains three types of sugars of D-glucose, D-fructose, and D-allulose, has functionality of D-allulose, and has a flavor similar to that of sugar, by combining the use of, for example, "inexpensive isomerized sugar or inverted sugar, which is a mixed sugar containing D-glucose and D-fructose" in term of a raw material cost occupying most of a manufacturing cost, the shortened multi-step process by production without using pseudo-moving layer chromatography, and the use of low-cost enzyme catalyst derived from high durability (lifetime) of the enzyme.

### BRIEF DESCRIPTION OF DRAWINGS

Fig. 1 is a diagram showing an influence of a temperature (optimum temperature) on a site-specific mutant enzyme of an immobilized Y586-1-derived ketose 3-epimerase.
Fig. 2 is a diagram showing an influence of a temperature (temperature stability) on the site-specific mutant enzyme of the immobilized Y586-1-derived ketose 3-epimerase.
Fig. 3 is a diagram showing an influence of a pH (optimum pH) on the site-specific mutant enzyme of the immobilized Y586-1-derived ketose 3-epimerase.
Fig. 4 is a diagram showing an influence of a pH (pH stability) on the site-specific mutant enzyme of the immobilized Y586-1-derived ketose 3-epimerase.
Fig. 5 is a drawing showing a table comparing various properties (optimum temperature, temperature stability, and optimum pH) of D-allulose 3-epimerase (purified enzyme) derived from Y586-1 (parent strain), a site-specific mutant enzyme (purified enzyme) of a Y586-1-derived D-allulose 3-epimerase, and the site-specific mutant enzyme (partially purified enzyme) of the immobilized Y586-1-derived D-allulose 3-epimerase.
Fig. 6 is a drawing for explaining a drying method of an immobilized enzyme.

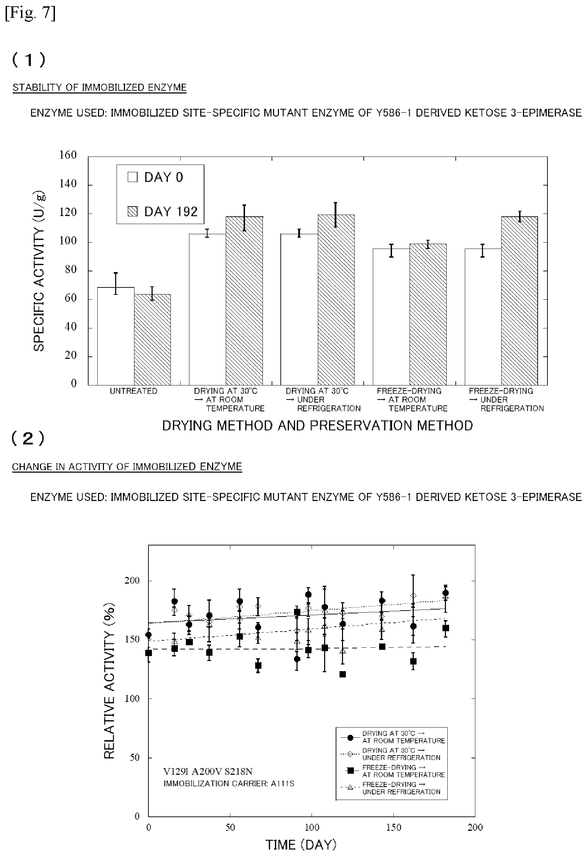

Fig. 7 (1) shows a comparison of activities of untreated and dried enzymes (drying at 30°C → preservation at room temperature, drying at 30°C → preservation under refrigeration (4°C), freeze-drying → preservation at room temperature, and freeze-drying → preservation under refrigeration (4°C)) on day 0 and day 192 of the preservation. Fig. 7 (2) shows a diagram showing the activity of the dried enzymes (drying at 30°C → preservation at room temperature, drying at 30°C → preservation under refrigeration (4°C), freeze-drying → preservation at room temperature, and freeze-drying → preservation under refrigeration (4°C)) as the relative activity with respect to 100% of the activity of the untreated enzyme.
Fig. 8 (1) shows a comparison of activities of untreated and dried enzymes (drying at 30°C → preservation at room temperature, drying at 30°C → preservation under refrigeration (4°C), freeze-drying → preservation at room temperature, and freeze-drying → preservation under refrigeration (4°C)) on day 0 and day 224 of the preservation.

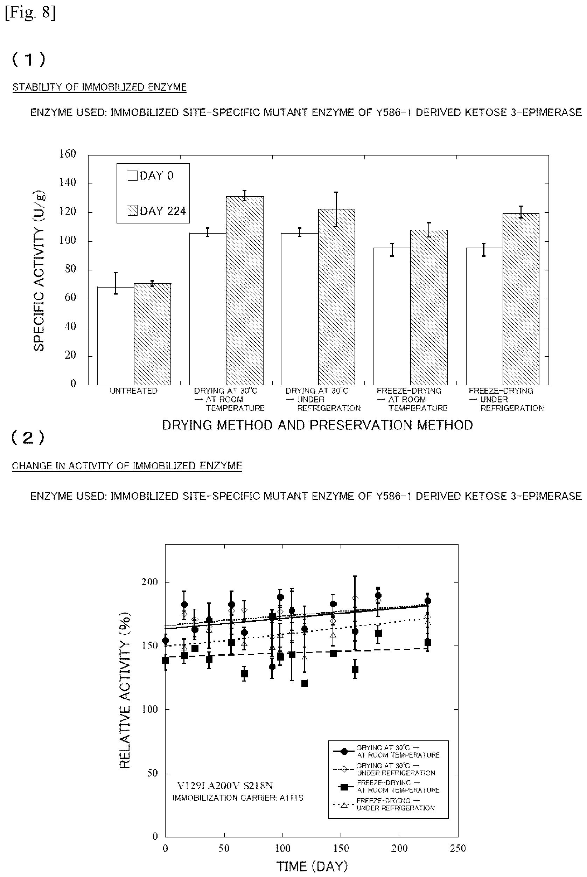

Fig. 8 (2) shows a diagram showing the activity of the dried enzymes (drying at 30°C → preservation at room temperature, drying at 30°C → preservation under refrigeration (4°C), freeze-drying → preservation at room temperature, and freeze-drying → preservation under refrigeration (4°C)) as the relative activity with respect to 100% of the activity of the untreated enzyme.

### DESCRIPTION OF EMBODIMENTS

### <Enzyme or protein having enzymatic activity thereof>

Currently, the rare sugar that can be produced in a large amount is D-psicose and D-allose. One of the English name notations of D-psicose is "D-allulose", and D-allulose is used in the present invention. D-allulose is a D-form of allulose classified into ketohexose, and is a six-carbon sugar (a hexose). In a case of considering the isomerase from the viewpoint of rare sugar production, the enzyme ketose 3-epimerase plays a part of the base enzyme.

An isomerase is a collective term for a group of enzymes that catalyze a reaction of converting a biological molecule into an isomer, and is also referred to as an isomerization enzyme. The term "isomerase" includes various enzyme groups that link aldose and ketose, which are a monosaccharide, and link ketose and ketose, and refers to an enzyme aldose isomerase that links aldose and ketose, and an enzyme ketose 3-epimerase that links ketose and ketose, which catalyze a "reaction in which a chemical formula does not change". Among the isomerases that catalyze the optical isomerism interconversion, an enzyme that isomerizes one of a plurality of chiral points <tertiary structure> in a substrate molecule is called an epimerase. An isomerization enzyme generally referred to as an epimerase because of the presence of a large number of chiral points in the sugar. In a case of monosaccharide, the epimerase is an enzyme that moves the position of -OH bonded to C to the opposite side, and catalyzes a "reaction in which chemical formula C₆H₁₂O₆ does not change". Therefore, epimerase is an enzyme belonging to an isomerase. The isomerase is present at a higher level than the epimerase.

That is, the enzyme "isomerase" is well known in the art, and examples thereof include a ketose 3-epimerase, D-xylose isomerase of an aldose isomerase, and a glucose isomerase. For example, an aldose isomerase catalyzes "movement of hydrogen within a molecule" and promotes an oxidation-reduction reaction between C₁ and C₂. D-tagatose 3-epimerase (DTE) which is a type of a ketose 3-epimerase is an enzyme found from a microorganism Pseudomonas cichorii that has been isolated from the campus of the Faculty of Agriculture, Kagawa University in 1991.
D-tagatose 3-epimerase inverts the orientation of the hydroxyl group at the carbon 3-position of D-tagatose to convert D-tagatose into D-sorbose. This reaction is a reversible equilibrium reaction, and the equilibrium ratio of D-tagatose and D-sorbose is 20:80. In addition, the present enzyme exhibits a wide substrate specificity, and can act not only on D-tagatose and D-sorbose but also on all ketoses having 6 carbon atoms, such as D-fructose and D-allulose (equilibrium ratio: 70:30), L-tagatose and L-sorbose (equilibrium ratio: 27:73), and L-allulose and L-fructose (equilibrium ratio: 24:76), and can also catalyze an epimerization reaction of ketoses having 5 carbon atoms, such as D-xylulose and D-ribulose (equilibrium ratio: 85:15) and L-xylulose and L-ribulose (equilibrium ratio: 70:30). In Kagawa University, it can be said that the discovery of the present enzyme has made it possible to produce a large number of rare sugars, and has been a driving force for the study of rare sugar production enzymes.

### <Dried body of immobilized enzyme or immobilized protein having enzymatic activity thereof, which is an enzyme capable of enduring drying, wherein enzyme or protein having enzymatic activity thereof is ketose 3-epimerase or protein having ketose 3-epimerase activity>

First, the ketose 3-epimerase or the protein having ketose 3-epimerase activity will be described.

In the present invention, the protein having ketose 3-epimerase activity preferably means an amino acid substitution mutant of a ketose 3-epimerase derived from Y586-1 strain, and is abbreviated as a site-specific mutant enzyme of a Y586-1 derived ketose 3-epimerase.

Among the rare sugars, D-allulose has been started to mass-produce on a global scale and put on the market, and is produced using a ketose 3-epimerase (for example, D-allulose 3-epimerase) with D-fructose as a raw material.

Therefore, D-allulose 3-epimerase (DAE) used for the production of D-allulose will be described as the enzyme to be dried.

The D-allulose 3-epimerase (DAE) may have any origin or classification name as long as it is an enzyme that isomerizes D-fructose into D-allulose.

For example, an Arthrobacter globiformis M30 strain, which is derived from a microorganism that is known to have no toxicity and that is allowed to be used in a case of manufacturing food, has high activity and heat resistance, and has been found to be able to catalyze the isomerization of D-fructose to D-allulose at high activity, is exemplified (Patent Document 5). The M30 strain has been originally deposited in Patent Microorganisms Depositary at National Institute of Technology and Evaluation in Japan (2-5-8 Kazusa-kamatari, Kisarazu-shi, Chiba, Japan) on June 22, 2011 with an accession number of NITE P-1111, and has been internationally deposited with accession number NITE BP-1111 according to the application of transfer to the deposition based on the Budapest Treaty on May 2, 2012.

In addition, a ketose 3-epimerase derived from Arthrobacter histidinolovorans Y586-1 strain is exemplified as a preferred (Patent Document 6).

The strain Y586-1 has been internationally deposited in Patent Microorganisms Depositary at National Institute of Technology and Evaluation located at Room No. 122, 2-5-8 Kazusa-kamatari, Kisarazu-shi, Chiba, Japan on November 7, 2018, and deposited with the accession number of NITE BP-02813.

### (Y586-1 strain)

The present inventors have focused on the strains which is listed in the list considered to have almost no toxicity, and are used as food not only in Japan but also in Europe and the United States, and have continued to search for a microorganism having ketose 3-epimerase activity by collecting soils from various places and isolating microorganisms from the soils. As a result, a microorganism belonging to the genus Arthrobacter that produces a novel ketose 3-epimerase was found in a large number of isolated strains. The Arthrobacter histidinolovorans Y586-1 strain, which is a microorganism of the genus Arthrobacter, produces a novel ketose 3-epimerase having high activity and high heat resistance.

The ketose 3-epimerase derived from the Y586-1 strain acts on free D- or L-ketose, and D- or L-ketopentose to epimerize the 3-position of these ketoses, and easily generates the corresponding D- or L-ketose and D- or L-ketopentose. This reaction leads the way for the mass production of D-allulose using various ketoses, particularly D-fructose, as a raw material.

In particular, the total activity obtained from the culture solution is about 3 times higher than that of the control enzyme as compared with the ketose 3-epimerase in the related art. Therefore, D-allulose can be mass produced by the enzyme derived from the Y586-1 strain.

In addition, since the optimum temperature of the enzyme is high as 70°C and the enzyme has high heat resistance, a higher yield of D-allulose production is obtained in a reaction at a temperature in the vicinity of 60°C to 70°C, which is the optimum temperature.

Furthermore, the amino acid substitution mutant of the ketose 3-epimerase derived from the Y586-1 strain include a large number of mutants having higher thermal stability than the present enzyme before mutation.

Based on the high activity and high heat resistance, the mutant is selected as a target of the dried immobilized enzyme to use the enzyme on a global scale.

This novel ketose 3-epimerase derived from this microorganism has an activity of acting on the 3-position of D- or L-ketose and catalyzing epimerization to the corresponding D- or L-ketose. Among D- or L-ketohexoses, the epimerization activity at a 3-position of D-allulose is highest, and thus it is suitable for manufacturing D-allulose from D-fructose.

That is, the ketose 3-epimerase selected as a target of the dried immobilized enzyme is a ketose 3-epimerase that can be preferably isolated from the microorganism belonging to the genus Arthrobacter, has a high enzyme productivity per culture solution, and has a characteristic property in thermal stability.

The microorganism of the genus Arthrobacter is a safe microorganism listed in the food additive list in Japan as an enzyme-origin microorganism such as α-amylase, isomaltodextranase, invertase, urease, glucanase, α-glucosyltransferase, and fructosyltransferase, or as a trehalose- or vitamin K (menaquinone)-producing bacterium. As an advantage of using the present enzyme derived from a microorganism of the genus Arthrobacter in the food industry, safety of the bacterium is mentioned.

Ketoses are a chemical term used in the case of classifying saccharides according to the structure thereof, and are a collective term for monosaccharides including one keto group (ketone carbonyl group) in the chain structure, and a molecular formula thereof is CₙH₂ₙOₙ (n ≥ 3). A representative ketose is fructose (fruit sugar), but also includes pentose and the like.

In the present invention, the ketose means a ketose in a broad sense including a pentose and the like, and in a case of being described as ketohexose, it means ketohexose which is a six-carbon sugar having a ketose structure. Specifically, the ketose is fructose, allulose, tagatose, or sorbose, and the D- or L-ketose means a D-form or L-form of these.

Therefore, the ketose 3-epimerase selected as a target of the dried immobilized enzyme according to the embodiment of the present invention has an activity of epimerizing the 3-position of a D- or L-ketose to generate a corresponding D- or L-ketose, and specifically, can catalyze the interconversion between D- or L-fructose and D- or L-allulose, and the interconversion between D- or L-tagatose and D- or L-sorbose. Furthermore, it can also act on all other ketoses.

As the ketose 3-epimerase derived from the Y586-1 strain, the produced enzyme has a higher activity by approximately twice per culture solution as compared with the ketose 3-epimerase derived from Arthrobacter globiformis described in Patent Document 5, D-allulose can be efficiently mass-produced by using the ketose 3-epimerase. In addition, an optimum temperature of the enzyme is high as 70°C, and a relative activity in a case of 80°C, in which the activity at the optimum temperature of 70°C is 100, is also 59.4, which is about 60%, and thus the enzyme has high heat resistance. Furthermore, since the residual activity after the incubation treatment at 60°C for 1 hour was 49.4%, which means that the enzyme had heat resistance, a larger amount of the product could be obtained in the reaction at 70°C as the optimum temperature. The enzyme has a characteristic that excellent heat resistance of the enzyme can be further enhanced by creating an amino acid substitution mutant of the enzyme.

That is, a mutant having various amino acid substitutions is prepared by introducing a mutation into the gene of the ketose 3-epimerase derived from the Y586-1 strain, to substitute a corresponding amino acid residue with another amino acid residue by a site-specific mutagenesis operation, thereby it is possible to obtain an enzyme having higher heat resistance than the wild-type enzyme that has not been subjected to amino acid substitution, and the mutant can be used. The mutant is a mutant having heat resistance, which has been evaluated for heat resistance by two indicators of a ratio (T70/T50) of the ketose 3-epimerase activity at a reaction temperature of 70°C to that at a reaction temperature of 50°C or residual activity after incubation at 60°C for 1 hour. As a result, a low-cost manufacturing method that is industrially practicable and is based on total productivity derived from high durability (lifetime) of the enzyme due to high heat resistance of the D-allulose 3-epimerase used has been realized.

Furthermore, various ketose 3-epimerase mutants are created by changing the base substitution by a site-specific mutagenesis operation, and in consideration of the general technical knowledge of enzymes that "an important property for increasing the lifetime of the enzyme is heat resistance", an amino acid substitution with which a mutant with higher heat resistance than that of the wild type is obtained is selected from among these mutants. 20 types of mutant enzymes that exceed the recombinant enzyme Y586-1 strain DAE (1.78) of the parent strain wild type are obtained among 37 types, and the 8th amino acid substitution mutant described in Table 10 in the specification of the international application (Patent Document 6) can be used.

The mutant is a protein represented by (1) a mutant sequence name and consisting of (2) an amino acid sequence set forth in SEQ ID NO: 1.
(1) Y586DAE_V129IA200VS218N
(2) amino acid sequence set forth in SEQ ID NO: 1

MKIGCHGLVWTGRFDAEGIRYSVQKTKEAGFDLIEFPLMDPFSFDVATAKSALAEHGLT
ASASLGLSEATDVSSEDPAIVKAGEELLNRALDVLAELGATDFCGVIYSAMKKYMEPAT
EQGLSNSKAAIGRVVDRAAGLGINVSLEVVNRYETNVLNTGRQALSYLSDVKRPNLGV
HLDTYHMNIEESDMFSPVLDTAEVLKYVHIGESHRGYLGTGNVDFDNFFKALGRIGYD
GPVVFESFSSAVVAPDLSRMLGIWRNLWADNEELGAHANAFIRDKLTAIKTIELH

By using such a mutant having heat resistance, total productivity derived from high durability (lifetime) of the enzyme rather than activity is provided from high heat resistance and high productivity. By using the ketose 3-epimerase or a protein having ketose 3-epimerase activity, the 3-position of the ketose which can be a substrate can be epimerized to convert the ketose into a corresponding ketose, and the corresponding ketose can be produced. Since the ketose 3-epimerase or the protein having ketose 3-epimerase activity has high epimerization activity at the 3-position with respect to D-allulose or D-fructose, it is possible to manufacture D-allulose efficiently and in a large amount, which is a rare sugar of the product, from D-fructose which can be a substrate.

In any case, as these enzymes, a purified enzyme may be used or a production microorganism of the enzyme may be used.

### <Immobilization of enzyme>

Next, the immobilized enzyme or protein in which the enzyme or protein having the enzymatic activity thereof is a ketose 3-epimerase or a protein having ketose 3-epimerase activity will be described. In addition, in the ketose 3-epimerase selected as a target of the dried immobilized enzyme according to the embodiment of the present invention, a highly active immobilized enzyme can be obtained by various immobilization methods, and the epimerization reaction can be continuously performed in large amounts by using the immobilized enzyme. By being industrially immobilized, the target ketose can be mass-produced.

Hereinafter, an enzyme may be used to meaning including a protein having enzymatic activity.

The enzyme is supported on a carrier which is an ion exchange resin, an organic polymer carrier, and/or an inorganic carrier, and is dried.

The immobilized enzyme itself is obtained by using a biological molecule, and is an immobilized enzyme in which the activity is inevitably decreased in a case of long-term use. However, it has been found that, in a case where the immobilized enzyme itself is in a stable state, the activity does not decrease even in a case of being dried. As these enzymes, a crude enzyme solution or a purified enzyme may be used, and a production microorganism or a protein having enzymatic activity may be used. The crude enzyme solution, the purified enzyme, production microorganism of the enzyme, or the protein having enzymatic activity is used in a state of being immobilized.

One aspect of a method of immobilizing a ketose 3-epimerase is immobilization using a crude enzyme solution. The ketose 3-epimerase can be immobilized by adding the crude enzyme solution containing the ketose 3-epimerase obtained by subjecting the suspension of bacterial cell to ultrasonic treatment to an ion exchange resin or the like and allowing the ketose 3-epimerase to be bound thereto at a low temperature.

In order to extract the ketose 3-epimerase from the bacterial cell, it is necessary to disrupt the bacterial cell wall, and there is a disrupting method by ultrasonic treatment, or enzyme treatment such as lysozyme, or the like. It has been found that the crude enzyme solution obtained by the ultrasonic treatment contains a large amount of the ketose 3-epimerase having activity.

The immobilized enzyme can be obtained by using a known immobilized means, for example, a carrier binding method, a crosslinking method, a gel encapsulation method, a microencapsulation method, or the like, and the carrier may be any carrier. In the carrier binding method, the enzyme can be bound (supported) to the carrier by physical adsorption, ion binding, covalent bonding, or the like.

In Examples, the enzyme was immobilized using a site-specific mutant enzyme of a ketose 3-epimerase derived from Arthrobacter histidinolovorans Y586-1 strain (parent strain). In Examples, immobilization is performed using a partially purified enzyme obtained by heat-treating a crude enzyme, but the properties of the immobilized enzyme are not changed even in the case of the crude enzyme or the purified enzyme, and the present invention is not limited to Examples.

By using the immobilized enzyme, it is possible to perform epimerization reaction continuously and in large amounts, and in a case where an enzyme having heat resistance derived from Arthrobacter histidinolovorans is used as the enzyme, the lifetime of the enzyme is extended. Any known carrier can be used for the carrier for immobilizing an enzyme, and it is most practical to immobilize the enzyme on an appropriate base material such as an ion exchange resin and a synthetic adsorbent. As the ion exchange resin, for example, a basic anion exchange resin, any of a strong basic anion exchange resin or a weak basic anion exchange resin can be used. Examples of the strong basic anion exchange resin include HPA25L (manufactured by Mitsubishi Chemical Corporation), IRA904CL (manufactured by ORGANO Corporation) and the like, and examples of the weak basic anion exchange resin include WA30 (manufactured by Mitsubishi Chemical Corporation), FPA54 and FPA95 (manufactured by ORGANO Corporation), and the like. In Examples, an anion exchange resin (A111S, manufactured by Purolite) is used for immobilization.

The isomerization by an enzyme can be performed using an immobilized enzyme, and a stable immobilized enzyme which is easily used can be obtained by various immobilization methods. By using the immobilized enzyme, it is possible to continuously perform a large amount of isomerization and isomerization reaction. For example, it is performed by using an immobilized enzyme having 1,000 U/wet weight resin (g) of activity. The crude enzyme solution is collected from the solution of the disrupted cells, which is obtained by disrupt the bacterial cell, and is allowed to flow through an ion exchange resin, a synthetic adsorbent, or the like, which is charged in a column, at a low temperature (4°C) to bind the crude enzyme protein to the ion exchange resin, and then a purified water is allowed to flow through the column to perform washing, thereby the immobilized enzyme can be obtained. An immobilized system which can withstand continuous production from the viewpoint of stability (maintenance of activity) that can be completely satisfied with commercial production is obtained. Furthermore, it goes without saying that an immobilized protein obtained by immobilizing an Escherichia coli-expressed enzyme may be used in the same manner as the crude enzyme and the purified enzyme.

Even after the obtained immobilized enzyme is dried and preserved for a long period of time, the isomerization reaction (epimerization reaction) can be performed continuously and in large amounts.

Since the immobilized ketose 3-epimerase can be easily eluted after the isomerization ability is lost, the immobilized carrier can be very easily regenerated, and the production cost can be reduced. In a case where an enzyme having heat resistance is used, the lifetime of the enzyme is extended, and the production cost can be further reduced. Even after drying and preservation, transportation, or the like, the enzyme can be easily returned to the state prior to drying, and can be used in the same manner as immediately after immobilization.

### <Confirmation of immobilized enzyme or protein having enzymatic activity thereof in which a temperature (an optimum temperature) indicating maximum activity has further improved as compared with prior to immobilization>

The activity is decreased by immobilization as compared with prior to immobilization. However, by being immobilized, the enzyme has an increase in the optimum temperature and stability to a long-term heat treatment.

An enzyme ketose 3-epimerase or a protein having ketose 3-epimerase activity is immobilized, the immobilized enzyme or the protein having enzymatic activity thereof in which a temperature (an optimum temperature) indicating maximum activity has further improved as compared with prior to immobilization is manufactured, and thereafter drying is performed.

Before drying, it has been confirmed by an experiment that the immobilized enzyme or the protein having enzymatic activity thereof is the immobilized enzyme or the protein having enzymatic activity thereof in which a temperature (an optimum temperature) indicating maximum activity has further improved as compared with prior to immobilization.

A method for measuring the enzymatic activity of the immobilized enzyme for the confirmation is shown as an example as follows.

The measurement of the enzymatic activity of the immobilized enzyme is performed by measuring the amount of D-fructose generated in a case where D-allulose is subjected to an enzyme reaction as a substrate.

First, a solution (400 µl) of 100 mg of the immobilized enzyme resin, a Tris-HCl buffer solution (pH 7.5) (final concentration of 50 mM), and D-allulose (final concentration of 100 mM) is used as a reaction solution composition, and the reaction solution composition is allowed to react at 30°C in a constant temperature water tank for 10 to 30 minutes, then boiled at 100°C for 2 minutes, and immediately the reaction is stopped. The solution after the reaction is cooled to room temperature, and then desalted with an ion exchange resin (a mixed resin of 200CT and IRA67 (both manufactured by Organo Corporation)), and further subjected to filter treatment to obtain an analysis sample. The analysis is performed by measuring the peak surface area of the generated D-fructose with high performance liquid chromatography (column: GL-C611 (Hitachi), temperature: 60°C, eluent: 0.1 mM NaOH, flow rate: 1.0 ml/min, detector: RID-20A (Shimadzu)).

The enzymatic activity depending on the carrier used for immobilizing the enzyme is compared, and an optimal carrier is selected.

In order to confirm the influence of the carrier used for enzyme immobilization, the enzymatic activities of the present enzyme immobilized on each carrier such as HPA25L, WA30, IRA904CL, FPA54, FPA95, and XAD7HP are compared.

The relative activity is obtained by calculating the sample having the largest peak surface area among the comparative samples as 100.

In selecting the produced immobilized enzyme having highest activity, the relative activity of the various immobilized enzymes is considered. In a case where the amount of the crude enzyme to be immobilized is increased, the relative activity in any resin is also increased.

In addition, the enzymatic activities of the immobilized enzymes are compared at different temperatures. A reaction is performed at various temperatures of 30°C to 80°C, and the relative activity of each carrier of various immobilized enzymes is measured. It can be seen that, regardless of whether or not the Mg²⁺ ion is added, the immobilized carrier should be selected according to the reaction temperature since the immobilized enzyme by the synthetic adsorbent XAD7HP has high relative activity at normal temperature, and the immobilized enzyme by the anion exchange resins HPA25L, IRA904CL, and WA30 has high relative activity at a high temperature.

### <Dried body>

Next, the dried body of the immobilized enzyme or protein in which the enzyme or protein having the enzymatic activity thereof is a ketose 3-epimerase or a protein having ketose 3-epimerase activity will be described.

The sample is a freeze-dried body or an incubator-dried body at 30°C. In a case where the immobilized enzyme is stable, the immobilized enzyme can maintain the activity in both drying method of the freeze-drying and the 30°C-incubator drying method.

In the dried body of the immobilized carrier, since the immobilized enzyme is also dried as well as the enzyme catalyst, the movement of the immobilized enzyme is also restricted, and thus the immobilized enzyme can be preserved for a long period of time. By the dehydration operation as drying, the enzyme molecule, which is originally stable in the presence of water molecules, can be unstable because of the loss of hydrogen bonding. However, it has been found that, rather, the enzyme molecule and the carrier molecule can form a hydrogen bond by the presence of an appropriate carrier, and the enzyme catalyst can stably exist even in a low moisture content, and that, despite the enzyme catalyst being a complex structural body, the (freezing) drying operation itself does not act as a denaturation factor in the immobilized form. A type of immobilized enzyme in which a ketose 3-epimerase (for example, D-allulose 3-epimerase) itself is carried on an ion exchange resin or the like can be adopted for preservation in a dry state.

It was found by experiments that, in a case where, in the immobilized enzyme, the stability having the same degree of activity as that before immobilization at the same temperature or a temperature higher than that the enzyme (ketose 3-epimerase) or the protein having enzymatic activity thereof (protein having ketose 3-epimerase activity) is a "dried body in which washing prior use is not required, transportation resistance is maintained, and the decrease in activity is suppressed" also after drying.

The preservation of the dried body is performed under refrigeration and at room temperature, and no significant decrease in activity is confirmed under both conditions. Therefore, there is no problem that the dried body is sent without being refrigerated.

Since the dried immobilized enzyme does not contain moisture, a paper container or a plastic container can be used for transportation. In a case of preservation in the experiment, a plastic container is used. Since it is not necessary to add a buffer solution or an additive, the cost for transporting the same amount of immobilized enzyme can be reduced as compared with a case where it is necessary to add a buffer solution or an additive. In addition, since only drying is performed, it is not necessary to remove the additives (washing the immobilized enzyme) before use. The dried immobilized enzyme is advantageous as a means for solving the problems regarding 1) price, 2) required number of days (several days to several months for marine transportation and 4 to 7 days for air transportation), and 3) temperature management (in a case where temperature management for maintaining quality is required, it is necessary to request a dedicated operator, and in a case of neglecting this, a temperature in a ship container passing through the equator may reach 70°C, and a container yard at a port or a transit point may also be at risk of being heated depending on the region and the season), which are elements for selecting a transportation means. The dried enzyme is realized by using enzyme capable of enduring drying.

### [Examples]

Specific examples of the present invention will be described with reference to Examples. The present invention is not limited to these Examples.

### (Drying of immobilized enzyme)

### <Culture of recombinant Escherichia coli>

The recombinant Escherichia coli highly expressing a mutant enzyme obtained by adding three site-specific mutations (V129I, A200V, and S218N) to the Y586-1-derived D-allulose 3-epimerase was cultured, and IPTG was added thereto to a final concentration of 1 mM to express the site-specific mutant enzyme. After the culturing, the recombinant Escherichia coli was collected by centrifugation.

### <Preparation of partially purified enzyme>

The obtained recombinant Escherichia coli was suspended in a 20 mM Tris-HCl buffer solution (pH 7.5) and the bacterial cells were disrupted using a beads crusher. After the treatment with the beads crusher, the centrifugation was performed, the cell residue was precipitated, and the supernatant containing the site-specific mutant enzyme was collected as a crude enzyme solution. In order to precipitate the nucleic acid, manganese chloride was added to the crude enzyme solution to have a concentration of 10 mM, and the solution was heat-treated at 60°C for 10 minutes to denature proteins other than the target enzyme and remove the proteins by centrifugation together with the nucleic acid. As a result, a partially purified enzyme was obtained.

### <Preparation of immobilized enzyme>

The obtained partially purified enzyme was added to an anion exchange resin (A111S) which had been equilibrated in advance with 20 mM Tris-HCl buffer solution (pH 7.5) to create an immobilized enzyme. The enzyme was immobilized on the carrier by adding a partially purified enzyme to the carrier such that the amount of the enzyme was 200 U per 1 g of the carrier, and appropriately stirring the mixture. After immobilization, the immobilized enzyme was washed with a 20 mM Tris-HCl buffer solution (pH 7.5) to prepare an immobilized enzyme.

### <Drying of immobilized enzyme>

Drying of the immobilized enzyme was performed at 30°C and by freeze-drying. The immobilized enzyme from which moisture had been removed in advance with a kimwipe was put into a container and dried.

The drying at 30°C was performed by allowing the immobilized enzyme to stand for 24 to 48 hours using an incubator to remove moisture.

The freeze-drying was performed by preliminarily freezing at -80°C and then freeze-drying for 24 to 48 hours to remove moisture.

### <Preservation of dried immobilized enzyme>

The dried immobilized enzyme was preserved at room temperature and 4°C.

### <Activity measurement of dried immobilized enzyme>

An appropriate amount of the preserved dried immobilized enzyme was weighed, and 700 µL of a 100 mM Tris-HCl buffer solution (pH 7.5) was added thereto. 700 µL of 200 mM D-allulose was further added as a substrate to start the enzyme reaction. The reaction was performed at a reaction temperature of 60°C for 10 minutes. After the reaction, the supernatant was collected and allowed to stand at 100°C for 3 minutes, and then filtered through a desalting treatment filter. The sugar composition in the reaction solution was analyzed using HPLC, and the activity was obtained.

The activity of the immobilized site-specific mutant enzyme of the Y586-1-derived ketose 3-epimerase in a reaction for 10 minutes was examined.

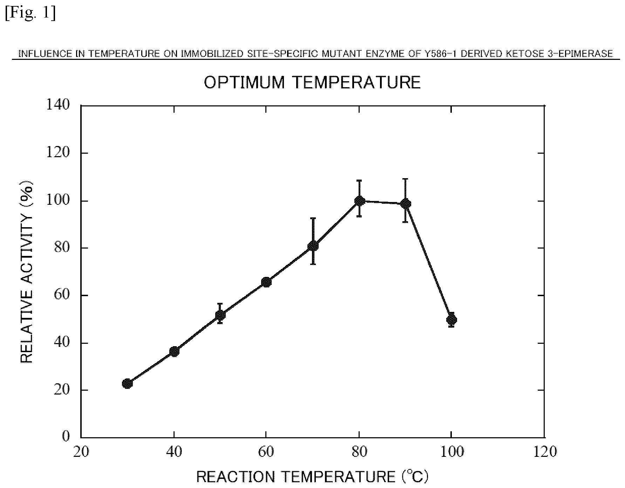

The immobilized enzyme obtained by immobilizing the site-specific mutant enzyme of Y586-1-derived ketose 3-epimerase was reacted at each temperature for 10 minutes, and the activity at that time was measured. As the substrate, D-allulose having a final concentration of 100 mM was used. As a buffer solution, a Tris-HCl buffer solution (pH 7.5) was used such that the final concentration was 50 mM. As a result, as shown in Fig. 1, the activity at 80°C and the activity at 90°C were the same degree, and the temperature exhibiting maximum activity was higher than the temperature at which the non-immobilized site-specific mutant enzyme of the Y586-1-derived ketose 3-epimerase exhibits the maximum activity.

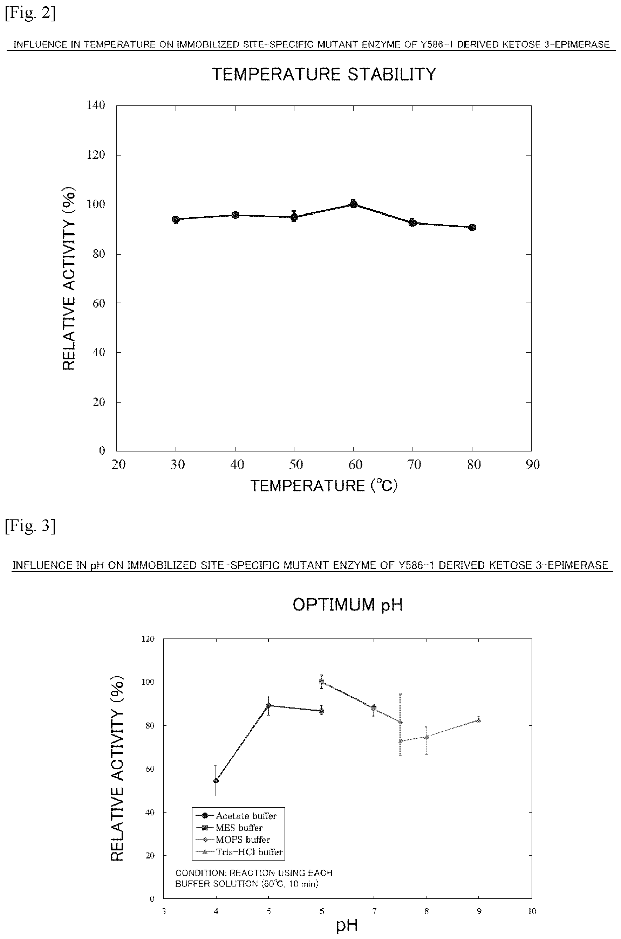

The immobilized site-specific mutant enzyme of the Y586-1-derived ketose 3-epimerase was heat-treated at each temperature for 60 minutes, and then cooled on ice. Thereafter, the reaction was performed at 60°C for 10 minutes, and the residual activity was measured. As a substrate, D-allulose having a final concentration of 100 mM was used. As a buffer solution, a Tris-HCl buffer solution (pH 7.5) was used such that the final concentration was 50 mM. As a result, as shown in Fig. 2, the decrease in activity was not confirmed up to 80°C, and the temperature stability was higher than that of the not-immobilized site-specific mutant enzyme of the Y586-1-derived ketose 3-epimerase. In the non-immobilized enzyme, the enzymatic activity was reduced even by the heat treatment for 10 minutes, but in the immobilized enzyme, the enzymatic activity was not reduced even by the heat treatment for 60 minutes, and the stability was high.

The activity of the immobilized site-specific mutant enzyme of the Y586-1-derived ketose 3-epimerase was examined by a reaction at 60°C for 10 minutes using each of the buffer solutions having different pH. As a substrate, D-allulose having a final concentration of 100 mM was used. Various buffer solutions were used such that the final concentration was 50 mM. As a result, as shown in Fig. 3, the pH exhibiting the maximum activity was pH 6.0 (MES buffer solution).

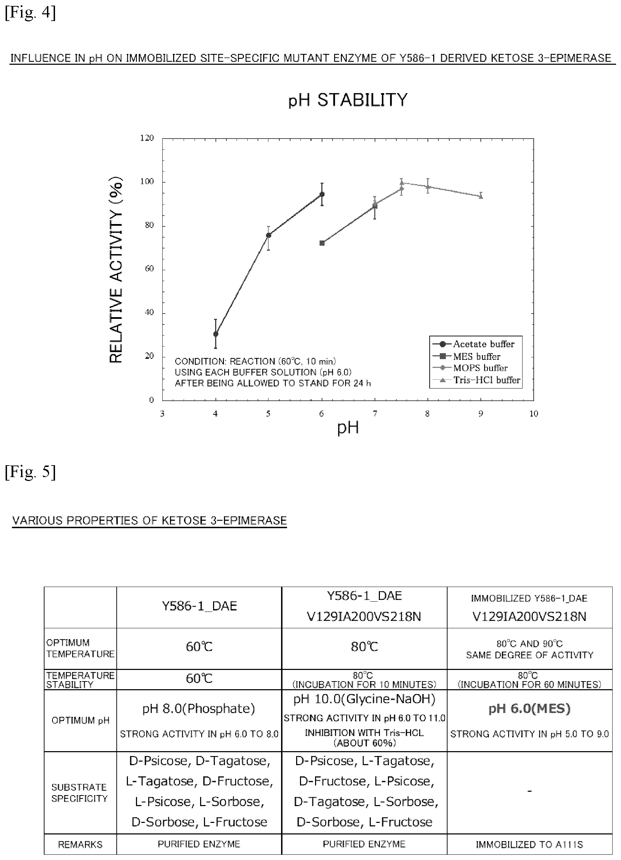

The pH stability of the immobilized site-specific mutant enzyme of the Y586-1-derived ketose 3-epimerase was examined. The immobilized enzyme obtained by immobilizing the site-specific mutant enzyme of the Y586-1-derived ketose 3-epimerase was allowed to stand in 100 mM each buffer solution for 24 hours, and then the buffer solution was removed and the enzyme was reacted at 60°C for 10 minutes using an MES buffer solution (pH 6.0) such that the final concentration was 50 mM, and the activity at that time was measured. As the substrate, D-allulose having a final concentration of 100 mM was used. As a result, as shown in Fig. 4, it was found that the enzyme had high activity at a pH of 5.0 to 9.0 and was stable.

Fig. 5 is a table comparing various properties (optimum temperature, temperature stability, and optimum pH) of D-allulose 3-epimerase (purified enzyme) derived from Y586-1 (parent strain), a site-specific mutant enzyme (purified enzyme) of a Y586-1-derived D-allulose 3-epimerase, and the site-specific mutant enzyme (partially purified enzyme) of the immobilized Y586-1-derived D-allulose 3-epimerase. It was confirmed that by the immobilization, the optimum temperature was increased, the temperature stability was improved, and the optimum pH was shifted to the acidic side.

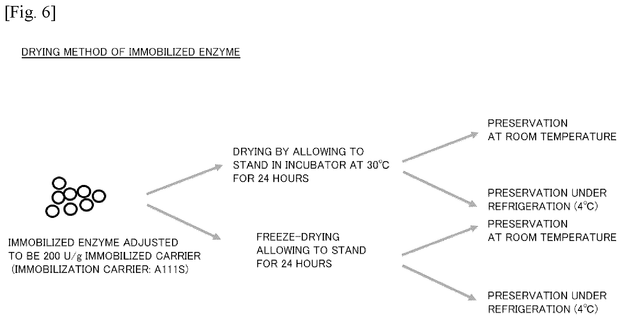

Fig. 6 is a diagram showing a drying method of the immobilized enzyme. The immobilized enzyme was produced by adding an enzyme, which had been partially purified by heat treatment at 60°C for 10 minutes, was added to the carrier such that the amount of enzyme is 200 U per 1 g of the carrier. The immobilized enzyme was dried in an incubator at 30°C or freeze-dried. The immobilized enzyme dried under each condition was preserved at room temperature or 4°C.

The enzymatic activity of the immobilized enzyme prior to drying and the immobilized enzyme dried under each condition (and preserved at room temperature and 4°C) was appropriately measured. The activity of the enzyme was examined by a reaction at 60°C for 10 minutes. As a substrate, D-allulose having a final concentration of 100 mM was used. As a buffer solution, a Tris-HCl buffer solution (pH 7.5) was used such that the final concentration was 50 mM. Fig. 7 (1) shows a comparison of activities of untreated and dried enzymes (drying at 30°C → preservation at room temperature, drying at 30°C → preservation under refrigeration (4°C), freeze-drying → preservation at room temperature, and freeze-drying → preservation under refrigeration (4°C)) on day 0 and day 192 of the preservation. There was no decrease in activity in all the samples. Fig. 7 (2) shows a diagram showing the activity of the dried enzymes (drying at 30°C → preservation at room temperature, drying at 30°C → preservation under refrigeration (4°C), freeze-drying → preservation at room temperature, and freeze-drying → preservation under refrigeration (4°C)) as the relative activity with respect to 100% of the activity of the untreated enzyme. The activity thereof was also not reduced.

Furthermore, the preservation period was extended by 1 month, and Fig. 8 (1) shows a comparison of activities of untreated and dried enzymes (drying at 30°C → preservation at room temperature, drying at 30°C → preservation under refrigeration (4°C), freeze-drying → preservation at room temperature, and freeze-drying → preservation under refrigeration (4°C)) on day 0 and day 224 of the preservation. There was no decrease in activity in all the samples. Fig. 8 (2) shows a diagram showing the activity of the dried enzymes (drying at 30°C → preservation at room temperature, drying at 30°C → preservation under refrigeration (4°C), freeze-drying → preservation at room temperature, and freeze-drying → preservation under refrigeration (4°C)) as the relative activity with respect to 100% of the activity of the untreated enzyme. The activity thereof was also not reduced.

### Industrial Applicability

Since the optimum temperature of the enzyme is high at 70°C and the enzyme has high heat resistance, in a reaction at a temperature in the vicinity of the optimum temperature of 60°C to 70°C, a ketose 3-epimerase having high activity and high heat resistance, or a site-specific mutant enzyme thereof, which are produced from the strain of Arthrobacter histidinolovorans Y586-1 with a higher yield of D-allulose production, can be transported in a form of a dried body after immobilization. Therefore, it is expected that a path for increasing production bases for mass production of D-allulose using D-fructose as a raw material in various countries around the world will be opened.

## Claims

1. A dried body of an immobilized enzyme or an immobilized protein having enzymatic activity thereof, which is an enzyme capable of enduring drying,
wherein the enzyme or the protein having enzymatic activity thereof is a ketose 3-epimerase or a protein having ketose 3-epimerase activity, and
the immobilized enzyme prior to drying or the immobilized protein having enzymatic activity thereof prior to drying is an immobilized enzyme or an immobilized protein having enzymatic activity thereof, in which a temperature (an optimum temperature) indicating maximum activity has further increased as compared with prior to immobilization.

2. The dried body of an immobilized enzyme or an immobilized protein having enzymatic activity thereof, which is an enzyme capable of enduring drying according to claim 1,
wherein the dried body is a dried body in which an additive for maintaining the enzymatic activity is not contained and the activity does not decrease for at least 6 months or more.

3. The dried body of an immobilized enzyme or an immobilized protein having enzymatic activity thereof, which is an enzyme capable of enduring drying according to claim 1 or 2,
wherein the dried body is a dried body of an enzyme or a protein having enzymatic activity thereof supporting by a carrier which is an ion exchange resin, organic polymer carrier, and/or an inorganic carrier.

4. The dried body of an immobilized enzyme or an immobilized protein having enzymatic activity thereof, which is an enzyme capable of enduring drying according to any one of claims 1 to 3,
wherein the dried body is a dried body in which washing prior use is not required, transportation resistance is maintained, and a decrease in activity is suppressed for at least 6 months or more.

5. The dried body of an immobilized enzyme or an immobilized protein having enzymatic activity thereof, which is an enzyme capable of enduring drying according to any one of claims 1 to 4,
wherein the dried body is a freeze-dried body or a 30°C-incubator-dried body.

6. The dried body of an immobilized enzyme or an immobilized protein having enzymatic activity thereof, which is an enzyme capable of enduring drying according to any one of claims 1 to 5,
wherein the enzyme or the protein having enzymatic activity thereof is selected from any one of the following enzyme (i) or protein having enzymatic activity thereof (ii),
(i) a ketose 3-epimerase derived from a microorganism belonging to the genus Arthrobacter, which has a molecular mass of 36 kDa measured by SDS-PAGE and has the following substrate specificities of (A) and (B) and the following physicochemical properties of (C) and (D),
(A) having activity of epimerizing a 3-position of a D- or L-ketose to generate a corresponding D- or L-ketose,
(B) having highest epimerization activity at a 3-position of a D-allulose among D- or L-ketohexoses,
(C) having a reaction optimum pH of 6 to 8,
(D) having a reaction optimum temperature of 60°C,
(ii) a protein described in the following (a), (b), (c), or (d),
(a) a protein consisting of an amino acid sequence set forth in SEQ ID NO: 1,
(b) a protein consisting of an amino acid sequence having a sequence identity of 75% or more with the amino acid sequence set forth in SEQ ID NO: 1, the amino acid sequence having no amino acid substitution at any sites of H6, S63, M110, E146, E152, D179, H182, H205, R211, or E240 of the amino acid sequence set forth in SEQ ID NO: 1, and maintaining a tertiary structure of the protein consisting of the amino acid sequence set forth in SEQ ID NO: 1, in which the protein has ketose 3-epimerase activity of the (A) and (B), and a ratio (T70/T50) of ketose 3-epimerase activity at a reaction temperature of 70°C to ketose 3-epimerase activity at a reaction temperature of 50°C is 1.55 or more,
(c) a protein consisting of an amino acid sequence having a sequence identity of 75% or more with the amino acid sequence set forth in SEQ ID NO: 1, the amino acid sequence having no amino acid substitution at any sites of H6, S63, M110, E146, E152, D179, H182, H205, R211, or E240 of the amino acid sequence set forth in SEQ ID NO: 1, and maintaining the tertiary structure of the protein consisting of the amino acid sequence set forth in SEQ ID NO: 1, in which the protein has the ketose 3-epimerase activity of the (A) and (B), and residual activity is 31% or more after incubation at 60°C for 1 hour,
(d) a protein consisting of an amino acid substitution mutant of the protein consisting of the amino acid sequence set forth in SEQ ID NO: 1, in which the protein has an amino acid substitution at at least one site of H56, T59, A60, P77, L94, L97, D101, A109, A128, V129, V132, S144, S167, P172, E199, A200, K202, or S218 of the amino acid sequence set forth in SEQ ID NO: 1, the protein has the ketose 3-epimerase activity of the (A) and (B), and the protein consists of a mutant having a higher ratio (T70/T50) of ketose 3-epimerase activity at a reaction temperature of 70°C to ketose 3-epimerase activity at a reaction temperature of 50°C or having a higher residual activity after incubation at 60°C for 1 hour as compared with the protein consisting of the amino acid sequence set forth in SEQ ID NO: 1.

7. The dried body of an immobilized enzyme or an immobilized protein having enzymatic activity thereof, which is an enzyme capable of enduring drying according to any one of claims 1 to 6,
wherein in the immobilized enzyme or the immobilized protein having enzymatic activity thereof, a crude enzyme present in a disrupted product of bacterial cell or a partially purified enzyme from a crude enzyme solution is immobilized on a carrier.

8. The dried body of an immobilized enzyme or an immobilized protein having enzymatic activity thereof, which is an enzyme capable of enduring drying according to any one of claims 1 to 7,
wherein substrate specificity of the ketose 3-epimerase is in the following order: D-allulose, D-tagatose, L-tagatose, D-fructose, and L-allulose.

9. The dried body of an immobilized enzyme or an immobilized protein having enzymatic activity thereof, which is an enzyme capable of enduring drying according to any one of claims 6 to 8,
wherein the microorganism belonging to the genus Arthrobacter is Arthrobacter histidinolovorans.

10. A manufacturing method of a dried body of an immobilized enzyme or an immobilized protein having enzymatic activity thereof, which is an enzyme capable of enduring drying, the manufacturing method comprising:
immobilizing an enzyme ketose 3-epimerase or a protein having protein ketose 3-epimerase activity;
manufacturing the immobilized enzyme or the protein having enzymatic activity of the enzyme in which a temperature (an optimum temperature) indicating maximum activity has further increased as compared with prior to immobilization; and
performing drying.

11. The manufacturing method of a dried body of an immobilized enzyme or an immobilized protein having enzymatic activity thereof, which is an enzyme capable of enduring drying according to claim 10,
wherein the enzyme ketose 3-epimerase or the protein having ketose 3-epimerase activity is supported by and immobilized on a carrier which is an ion exchange resin, an organic polymer carrier, and/or an inorganic carrier.

12. The manufacturing method of a dried body of an immobilized enzyme or an immobilized protein having enzymatic activity thereof, which is an enzyme capable of enduring drying according to claim 10 or 11,
wherein the drying is performed without adding an additive for maintaining the enzymatic activity.

13. The manufacturing method of a dried body of an immobilized enzyme or an immobilized protein having enzymatic activity thereof, which is an enzyme capable of enduring drying according to any one of claims 10 to 12,
wherein the drying is performed by freeze-drying or incubator-drying at 30°C.

14. The manufacturing method of a dried body of an immobilized enzyme or an immobilized protein having enzymatic activity thereof, which is an enzyme capable of enduring drying according to any one of claims 10 to 13,
wherein the enzyme ketose 3-epimerase or the protein having ketose 3-epimerase activity is selected from any one of the following enzyme (i) or protein having enzymatic activity of the enzyme (ii),
(i) a ketose 3-epimerase derived from a microorganism belonging to the genus Arthrobacter, which has a molecular mass of 36 kDa measured by SDS-PAGE and has the following substrate specificities of (A) and (B) and the following physicochemical properties of (C) and (D),
(A) having activity of epimerizing a 3-position of a D- or L-ketose to generate a corresponding D- or L-ketose,
(B) having highest epimerization activity at a 3-position of a D-allulose among D- or L-ketohexoses,
(C) having a reaction optimum pH of 6 to 8,
(D) having a reaction optimum temperature of 60°C,
(ii) a protein described in the following (a), (b), (c), or (d),
(a) a protein consisting of an amino acid sequence set forth in SEQ ID NO: 1,
(b) a protein consisting of an amino acid sequence having a sequence identity of 75% or more with the amino acid sequence set forth in SEQ ID NO: 1, the amino acid sequence having no amino acid substitution at any sites of H6, S63, M110, E146, E152, D179, H182, H205, R211, or E240 of the amino acid sequence set forth in SEQ ID NO: 1, and maintaining a tertiary structure of the protein consisting of the amino acid sequence set forth in SEQ ID NO: 1, in which the protein has ketose 3-epimerase activity of the (A) and (B), and a ratio (T70/T50) of ketose 3-epimerase activity at a reaction temperature of 70°C to ketose 3-epimerase activity at a reaction temperature of 50°C is 1.55 or more,
(c) a protein consisting of an amino acid sequence having a sequence identity of 75% or more with the amino acid sequence set forth in SEQ ID NO: 1, the amino acid sequence having no amino acid substitution at any sites of H6, S63, M110, E146, E152, D179, H182, H205, R211, or E240 of the amino acid sequence set forth in SEQ ID NO: 1, and maintaining the tertiary structure of the protein consisting of the amino acid sequence set forth in SEQ ID NO: 1, in which the protein has the ketose 3-epimerase activity of the (A) and (B), and residual activity is 31% or more after incubation at 60°C for 1 hour,
(d) a protein consisting of an amino acid substitution mutant of the protein consisting of the amino acid sequence set forth in SEQ ID NO: 1, in which the protein has an amino acid substitution at at least one site of H56, T59, A60, P77, L94, L97, D101, A109, A128, V129, V132, S144, S167, P172, E199, A200, K202, or S218 of the amino acid sequence set forth in SEQ ID NO: 1, the protein has the ketose 3-epimerase activity of the (A) and (B), and the protein consists of a mutant having a higher ratio (T70/T50) of ketose 3-epimerase activity at a reaction temperature of 70°C to ketose 3-epimerase activity at a reaction temperature of 50°C or having a higher residual activity after incubation at 60°C for 1 hour as compared with the protein consisting of the amino acid sequence set forth in SEQ ID NO: 1.

15. The manufacturing method of a dried body of an immobilized enzyme or an immobilized protein having enzymatic activity thereof, which is an enzyme capable of enduring drying according to any one of claims 10 to 14,
wherein a crude enzyme present in a disrupted product of bacterial cell or a partially purified enzyme from a crude enzyme solution is immobilized on a carrier.

16. The manufacturing method of a dried body of an immobilized enzyme or an immobilized protein having enzymatic activity thereof, which is an enzyme capable of enduring drying according to any one of claims 10 to 15,
wherein substrate specificity of the ketose 3-epimerase is in the following order: D-allulose, D-tagatose, L-tagatose, D-fructose, and L-allulose.

17. The manufacturing method of a dried body of an immobilized enzyme or an immobilized protein having enzymatic activity thereof, which is an enzyme capable of enduring drying according to any one of claims 14 to 16,
wherein the microorganism belonging to the genus Arthrobacter is Arthrobacter histidinolovorans.

18. The manufacturing method of a dried body of an immobilized enzyme or an immobilized protein having enzymatic activity thereof, which is an enzyme capable of enduring drying according to any one of claims 14 to 17,
wherein the microorganism belonging to the genus Arthrobacter is Arthrobacter histidinolovorans Y586-1 strain deposited in Patent Microorganisms Depositary with accession number NITE BP-02813.

19. A preservation method of the dried body of an immobilized enzyme or an immobilized protein having enzymatic activity thereof, which is an enzyme capable of enduring drying according to any one of claims 1 to 9, the preservation method comprising:
performing preservation at any one of a refrigerated temperature or a room temperature.

20. The preservation method of the dried body of an immobilized enzyme or an immobilized protein having enzymatic activity thereof, which is an enzyme capable of enduring drying according to claim 19,
wherein the dried body is sealed in a sealable container consisting of paper, plastic, or metal.
